# EUROPÄISCHE PATENTSCHRIFT

(11) **EP 2 486 885 B1**
(45) Veröffentlichungstag und Bekanntmachung des Hinweises auf die Patenterteilung: **01.05.2013**
(21) Anmeldenummer: 11153757.7
(22) Anmeldetag: 09.02.2011
(51) Int. Cl.: A61B 18/14, A61B 18/12, H01R 13/10

(54) **Universalsteckbuchse**
Universal slide-on
Fiche femelle universelle

(43) Veröffentlichungstag der Anmeldung: 15.08.2012
(73) Patentinhaber: ERBE Elektromedizin GmbH, 72072 Tübingen (DE)
(72) Erfinder: Selig, Peter, 72379, Hechingen (DE); Hundt, Roland, 72810, Gomaringen (DE)
(74) Vertreter: Bohnenberger, Johannes

(56) Entgegenhaltungen:
- DE-A1-102009 022 363
- US-A- 5 573 424
- US-A1- 2003 078 572

## Beschreibung

Die vorliegende Erfindung betrifft eine Universalsteckbuchse für ein Chirurgiegerät gemäß dem Oberbegriff des Anspruchs 1.

Eine Universalsteckbuchse der hier angesprochenen Art ist beispielsweise aus der DE 10 2007 061 483 A1 bekannt. Sie dient zum Einbau in ein Chirurgiegerät und zum Anschluss von unterschiedlichen Steckverbindern, die verschiedenen elektrochirurgischen Instrumenten zum Einsatz in der Hochfrequenz-Chirurgie zugeordnet sein können. Die Universalsteckbuchse weist mehrere Steckkontaktöffnungen zur Aufnahme von Kontaktstiften eines jeweiligen Steckverbinders auf, der mit einem beliebigen elektrochirurgischen Instrument verbunden ist. Über die Steckkontaktöffnungen und die Kontaktstifte wird dem elektrochirurgischen Instrument ein geeigneter hochfrequenter Strom zugeführt, wobei der notwendige HF-Strom üblicherweise durch einen in dem Chirurgiegerät befindlichen HF-Generator erzeugt wird. Aus dem Stand der Technik sind weiterhin viele verschiedene Steckverbinder zum Anschluss von Zubehör, wie beispielsweise chirurgische Handgriffe oder Pinzetten an ein HF-Chirurgiegerät, bekannt. Üblicherweise unterscheiden sich diese Instrumente untereinander in funktioneller Hinsicht höchstens geringfügig. Allerdings kann die mechanische Ausgestaltung der zugehörigen Steckverbinder durchaus unterschiedlich sein. Jeder Hersteller hat nämlich bevorzugte Steckverbinder im Programm, sodass die Steckverbinder identischer chirurgischer Instrumente, die aber von unterschiedlichen Herstellern stammen, voneinander abweichen können. Zur Verbindung mit gleichen Instrumententypen sind dann unterschiedliche Steckbuchsen notwendig. Auf der anderen Seite haben sich im Laufe der Zeit einige besonders vorteilhafte Steckverbindertypen herauskristallisiert, die von allen Herstellern angeboten werden. Prinzipiell wird dabei zwischen rein bipolaren und rein monopolaren Steckbuchsentypen unterschieden, d.h. also zwischen Steckbuchsen zum Anschluss von monopolaren Instrumenten mit einer aktiven Elektrode und Steckbuchsen zum Anschluss von bipolaren Instrumenten mit zwei aktiven Elektroden. Der monopolare "3-Pin"-Steckverbinder bildet beispielsweise einen oben angesprochenen bevorzugten monopolaren Steckverbindertyp, der auch von Drittanbietern für Zubehör genutzt wird. Im bipolaren Bereich stehen zwei bevorzugte Steckverbinder zur Verfügung, die je zwei Kontaktstifte aufweisen, wobei die Kontaktstifte bei dem einen Steckverbinder einen Abstand von 22 mm und bei dem anderen Steckverbinder 28,5 mm aufweisen. Für einen kleinen Bereich von Steckverbindern gibt es bereits Universalsteckbuchsen, die verschiedene Steckverbinder aufnehmen können. Allerdings beschränken sich diese jeweils auf den rein bipolaren oder rein monopolaren Bereich. Auch in ihrer Funktionalität sind diese bekannten Steckbuchsen stark eingeschränkt. Üblicherweise handelt es sich um Steckverbinder, die ausschließlich mechanisch universelle Baugruppen bilden.

Aufgabe der vorliegenden Erfindung ist es daher, eine universelle Steckverbindung sowie eine Universalsteckbuchse für ein Chirurgiegerät zu schaffen, welche einerseits eine verbesserte mechanische universelle Einsetzbarkeit aufweist und darüber hinaus eine erweiterte Funktionalität bieten kann.

Zur Lösung der oben genannten Aufgabe wird eine Universalsteckbuchse mit den Merkmalen des Anspruchs 1 sowie eine universelle Steckverbindung mit den Merkmalen des Anspruchs 12 vorgeschlagen. Sie zeichnet sich dadurch aus, dass zum wahlweisen Anschluss von monopolaren und bipolaren chirurgischen Instrumenten mehrere Steckkontaktöffnungen überlappend angeordnet sind, und dass zur Festlegung einer eindeutigen Steckposition eines Steckverbinders eine gemeinsame Bezugssteckkontaktöffnung für unterschiedliche Steckverbinder vorgesehen ist, die bezüglich der anderen Steckkontaktöffnungen getrennt angeordnet ist.

Ein wesentlicher Punkt der Erfindung liegt darin, eine universelle Steckverbindung bereitzustellen, welche sowohl marktübliche monopolare als auch bipolare Steckverbinder aufnehmen kann und funktionell auf die eingesetzten Steckverbinder reagiert. Dazu umfasst die Stechverbindung wenigstens ein Kontaktmittel in Form einer Öffnung, die der Universalsteckbuchse zugeordnet ist und wenigstens ein vorstehendes Kontaktelement in Form eines Vorsprungs beispielsweise eines Stiftes der dem Steckverbinder zugeordnet ist.

Darüber hinaus bildet die gemeinsame Bezugssteckkontaktöffnung für unterschiedliche Steckverbinder eine eindeutige Steckerposition, wobei durch den festen Abstand der Kontaktelemente eines Steckerverbinders zueinander eine eindeutige Steckposition garantiert wird. Es wird folglich eine mechanische Steckbuchse vorgeschlagen, die sowohl runde als auch ovale bzw. längliche Steckkontaktöffnungen umfasst, um die Vielzahl an Kontaktabständen der unterschiedlichen Steckverbinder abdecken zu können.

Vorzugsweise weist zumindest eine Steckkontaktöffnung der Universalsteckbuchse mehrere elektrisch voneinander getrennte Kontaktbereiche auf, die zur Verbindung mit einem Kontaktelement dienen, das ebenfalls mehrere Kontaktbereiche aufweist. Wenn das Kontaktelement in die Steckkontaktöffnung eingesteckt ist, berühren sich die Kontaktbereiche des Kontaktelements und der Steckkontaktöffnung und bilden elektrisch voneinander getrennte bzw. isolierte Strom- und/oder Signalwege. Die mehreren elektrisch getrennten Kontaktbereiche der zumindest einen Steckkontaktöffnung können unterschiedlich ausgebildet und angeordnet sein. So ist es möglich, dass die Steckkontaktöffnung mehrere in axialer Richtung elektrisch getrennte Kontaktbereiche an ihrem Innenumfang aufweist und somit mit einem Kontaktelement nach Art eines Klinkensteckers zusammenwirken kann. Es ist auch möglich, dass die mehreren Kontaktbereiche der zumindest einen Steckkontaktöffnung in Längsrichtung an ihrem Innenumfang elektrisch getrennt voneinander angeordnet sind und mit einem Kontaktelement, dessen Kontakte auch in Längsrichtung elektrisch getrennt angeordnet sind, zusammen wirken kann. Die Kontaktbereiche können über die gesamte Länge eines Kontaktelements bzw. der entsprechenden Steckkontaktöffnung angeordnet sein. Sie können aber auch nur einen Teil dieser Länge einnehmen und elektrisch isolierend hintereinander und zusätzlich nebeneinander am Umfang des Kontaktelements bzw. der Steckkontaktöffnung angeordnet sein. Es ist auch möglich, dass ein Kontaktelement mehrere einzeln elektrisch voneinander isolierte Kontaktstifte aufweist. Dann weist die Steckkontaktöffnung eine der Kontaktstifte entsprechende Anzahl Kontaktbuchsen auf um eine elektrische Kontaktierung wirksam sicherzustellen. Die Kontaktelemente bzw. die Kontaktstifte können runde, ovale, quadratische, rechteckige oder auch davon abweichende Formen aufweisen, wobei dann die dem Kontaktelement oder dem Kontaktstift zugeordnete Steckkontaktöffnung eine korrespondierende Querschnittsform aufweist damit beim Zusammenführen des Kontaktelements und der Steckkontaktöffnung eine elektrische Wirkverbindung entsteht. Es ist auch möglich, dass eine elektrische Kontaktierung zwischen einer Steckkontaktöffnung und einem Kontaktelement aus einer Kombination von unterschiedlich ausgebildeten Kontaktbereichen, wie vorstehend beschrieben, umfasst. Weiterhin kann vorgesehen sein, dass die Universalsteckbuchse gemäß der vorliegenden Erfindung wenigstens eine Steckerkennungseinrichtung aufweist, so dass erkannt wird, wenn sich ein Steckverbinder in der Universalsteckbuchse befindet. Eine Steckerkennungseinrichtung kann durch wenigstens einen zusätzlichen Kontaktpin, durch wenigstens einen Reed-Kontakt oder durch wenigstens einen Mikroschalter realisiert sein. Weiterhin kann eine Instrumentenerkennungseinrichtung zur Erkennung des mit der Universalsteckbuchse verbundenen chirurgischen Instrumententyps vorgesehen sein. Vorzugsweise sind wenigstens zwei Steckkontaktöffnungen in der Universalsteckbuchse vorgesehen, die einen Abstand von 22 mm zueinander aufweisen. Vorzugsweise sind noch zwei weitere Steckkontaktöffnungen vorgesehen, die einen Abstand von 28,5 mm zueinander aufweisen. Auf diese Weise kann die Universalsteckbuchse bipolare Steckverbinder aufnehmen, die Kontaktelemente mit unterschiedlichen Abständen aufweisen. Bei einer besonders bevorzugten Ausführungsform sind insgesamt fünf Steckkontaktöffnungen vorgesehen, die entlang einer gemeinsamen Mittelachse angeordnet sind.

Dokument US-2003/078572 offenbart den nächstligenden Stand der Technik.

Die Erfindung wird im Folgenden anhand der Zeichnung näher erläutert.

Es zeigen:
- Fig. 1: eine schematische Draufsicht auf eine herkömmliche 3-Pin Steckbuchse zum Anschluss eines monopolaren elektrochirurgischen Instruments;
- Fig. 2: eine schematische Draufsicht auf eine herkömmliche 2-Pin Steckbuchse eines ersten Typs zum Anschluss eines bipolaren elektrochirurgischen Instruments;
- Fig. 3: eine schematische Draufsicht auf eine herkömmliche 2-Pin Steckbuchse eines zweiten Typs zum Anschluss eines bipolaren elektrochirurgischen Instruments;
- Fig. 4: eine schematische Draufsicht auf eine Universalsteckbuchse gemäß der Erfindung zum Anschluss eines monopolaren und eines bipolaren elektrochirurgischen Instruments;
- Fig. 5: eine schematische Seitenansicht eines multifunktionalen 3-Pin Steckverbinders für ein monopolares elektrochirurgisches Instrument;
- Fig. 6: eine schematische Darstellung eines einfachen 3-Pin Steckverbinders für ein monopolares elektrochirurgisches Instrument;
- Fig. 7A: eine schematische Darstellung eines einfachen/multifunktionalen 3-Pin Steckverbinders für ein monopolares elektrochirurgisches Instrument;
- Fig. 7B: eine schematische Darstellung eines ersten einfachen/multifunktionalen 2-Pin Steckverbinders für ein bipolares elektrochirurgisches Instrument;
- Fig. 7C: eine schematische Darstellung eines zweiten einfachen/multifunktionalen 2-Pin Steckverbinders für ein bipolares elektrochirurgisches Instrument, und
- Fig. 7D: eine schematische Schnittdarstellung einer Universalsteckbuchse gemäß der Erfindung.

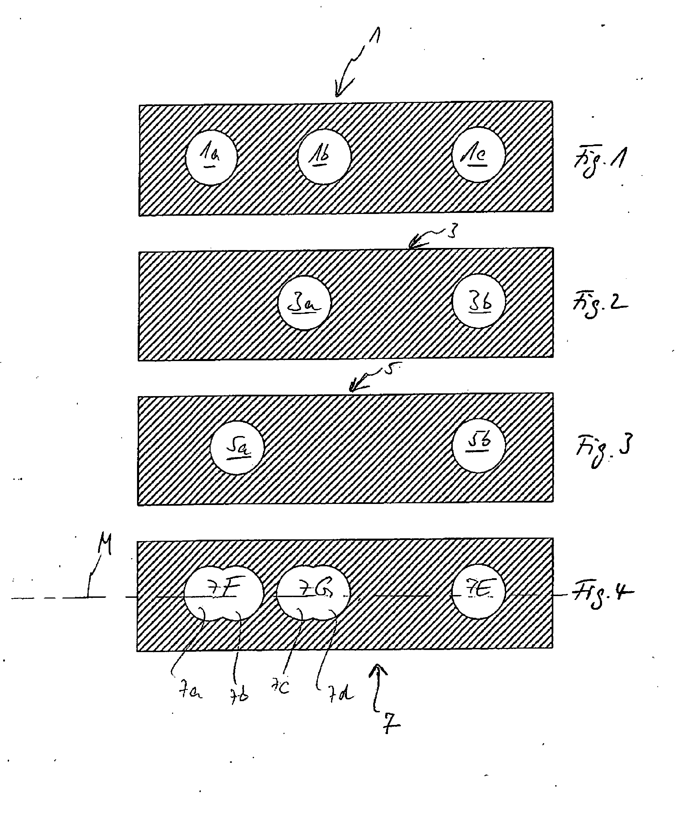

Fig. 1 zeigt eine schematische Draufsicht auf eine herkömmliche 3-Pin Steckbuchse 1 zum Anschluss eines monopolaren, in der Figur nicht gezeigten, elektrochirurgischen Instruments. Die Steckbuchse 1 ist hier isoliert dargestellt, es versteht sich aber, dass sie üblicherweise in ein Chirurgiegerät von außen zugänglich integriert ist. Die Steckbuchse 1 weist insgesamt drei Steckkontaktöffnungen 1a, 1b und 1c zur Aufnahme von Kontaktelementen, welche in diesem Ausführungsbeispiel in Form von Kontaktstiften dargestellt sind, eines Steckverbinders auf, wobei der Steckverbinder mit einem beliebigen chirurgischen Instrument verbunden sein kann. Die Tiefe der Steckkontaktöffnungen entspricht vorzugsweise der Länge der Kontaktelemente, sodass die Kontaktelemente vorzugsweise bis zum Anschlag in die Steckkontaktöffnungen eingesteckt werden können. Eine elektrische Verbindung zwischen den Steckkontaktöffnungen und den Kontaktelementen kommt über Kontakte zu Stande, die einerseits an der Innenwandung der Steckkontaktöffnungen und andererseits an der Umfangsfläche der Kontaktelemente vorgesehen sind und die sich im gesteckten Zustand der Kontaktelemente in den Steckkontaktöffnungen berühren. Über die elektrische Verbindung zwischen den Kontakten der Steckkontaktöffnungen 1a, 1b und 1c und den Kontaktelementen des Steckverbinders wird dem elektrochirurgischen Instrument ein hochfrequenter Strom von einem HF-Generator zugeführt.

Fig. 2 zeigte eine herkömmliche Steckbuchse 3 zum Anschluss eines bipolaren chirurgischen Instruments. Die Buchse weist zwei Steckkontaktöffnungen 3a und 3b auf, die in einem Abstand von 22,0 mm zueinander angeordnet sind und zur Aufnahme eines entsprechenden Steckverbinders dienen. Ein geeigneter Steckverbinder weist folglich zwei passende Kontaktelemente auf, die ebenfalls in einem Abstand von 22,0 mm zueinander angeordnet sind.

Fig. 3 zeigt eine dritte herkömmliche Steckbuchse 5, die ebenfalls zum Anschluss eines bipolaren elektrochirurgischen Instruments dient. Die Steckbuchse 5 umfasst zwei Steckkontaktöffnungen 5a und 5b, die im Gegensatz zu der Steckbuchse 3 gemäß Fig. 2 in einem Abstand von 28,5 mm zueinander angeordnet sind. Die Steckbuchse 5 dient zur Aufnahme eines entsprechenden Steckverbinders, der zwei Kontaktelemente aufweist, die ebenfalls in einem Abstand von 28,5 mm zueinander angeordnet sind.

Die oben zu den Fig. 1 bis 3 beschriebenen herkömmlichen Steckbuchsen bilden besonders bevorzugte Steckbuchsen, die praktisch von jedem Herstellen von elektromedizinischen Geräten oder von elektromedizinischem Zubehör angeboten werden. Herkömmlicherweise sind die Steckbuchsen einzeln in ein Chirurgiegerät integriert, sodass je nach Art des Steckverbinders, die monopolare oder eine bipolare Steckbuchse zum Anschluss eines elektrochirurgischen Instruments verwendet werden kann.

Die Fig. 4 zeigt eine schematische Draufsicht auf eine Universalsteckbuchse 7 gemäß der vorliegenden Erfindung, welche erfindungsgemäß die bekannten bevorzugten monopolaren und bipolaren Steckbuchsen in einer einzigen Universalsteckbuchse vereint. Die Universalsteckbuchse 7 dient also zum wahlweisen Anschluss von monopolaren und bipolaren elektrochirurgischen Instrumenten. Im Gegensatz zu den herkömmlichen Steckbuchsen weist sie mehrere Steckkontaktöffnungen 7a, 7b, 7c, 7d auf, die entlang einer Mittelachse M der Universalsteckbuchse 7 zumindest teilweise überlappend angeordnet sind. Darüber hinaus ist eine weitere kreisrunde Steckkontaktöffnung 7E vorgesehen, die getrennt von den anderen Steckkontaktöffnungen, also nicht überlappend mit einer anderen Steckkontaktöffnung angeordnet ist.

Vorzugsweise sind wie in der Fig. 4 dargestellt, jeweils zwei der Steckkontaktöffnungen, nämlich 7a und 7b sowie 7c und 7d, entlang der Mittelachse M überlappend angeordnet. Die Steckkontaktöffnungen überlappen sich also paarweise. Auf diese Weise resultieren zwei der Steckkontaktöffnungen 7a und 7b in einer im Wesentlichen ovalen bzw. länglichen (entlang der Mittelachse M erstreckenden) gemeinsamen Steckkontaktöffnung 7F und die beiden anderen überlappenden Steckkontaktöffnungen 7c und 7d resultieren ebenfalls in einer sich entlang der Mittelachse M erstreckenden länglichen bzw. im Wesentlichen ovalen gemeinsamen Steckkontaktöffnung 7G. Sämtliche Steckkontaktöffnungen 7a, 7b, 7c, 7d sowie die separate Steckkontaktöffnung 7E sind vorzugsweise entlang der gemeinsamen Mittelachse M der Universalsteckbuchse 7 angeordnet. Grundsätzlich denkbar ist es jedoch auch, wenigstens einige der Steckkontaktöffnungen 7a, 7b, 7c, 7d auf konzentrischen Kreisen anzuordnen, die als gemeinsamen Mittelpunkt die Steckkontaktöffnung 7E aufweisen. Platzsparender und kostengünstiger ist jedoch die Variante, bei der die Steckkontaktöffnungen einander paarweise überlappen.

Im vorliegenden Beispiel entspricht die Position der Steckkontaktöffnungen 7a, 7d und 7E und der Abstand zwischen diesen Steckkontaktöffnungen der Position und dem Abstand zwischen den Steckkontaktöffnungen 1a, 1b und 1c der monopolaren 3-Pin Steckbuchse aus Fig. 1. Die Steckkontaktöffnungen 7c und 7E sind weiterhin so angeordnet, dass ihre Position und ihr Abstand zueinander den Steckkontaktöffnungen 3a und 3b der bipolaren 22,0 mm 2-Pin Steckbuchse aus Fig. 2 entsprechen. Die Steckkontaktöffnungen 7b und 7E sind schließlich so angeordnet, dass ihre Position und ihr Abstand zueinander den Steckkontaktöffnungen 5a und 5b der bipolaren 28,5 mm 2-Pin Steckbuchse aus Fig. 2 entsprechen. Es versteht sich, dass zum Verbinden mit Steckverbindern, die einen anderen Abstand zwischen ihren Kontaktelementen aufweisen als es in den vorliegenden Beispielen der Fall ist, auch jeweils mehr als zwei Steckkontaktöffnungen überlappend angeordnet sein können bzw. die Abstände zwischen den Steckkontaktöffnungen variieren können.

Die Steckkontaktöffnung 7E bildet eine gemeinsame Bezugssteckkontaktöffnung für unterschiedliche Steckverbinder. D.h., dass einer der Kontaktelemente jedes Steckverbinders, der mit der Universalsteckbuchse verbunden wird, in jedem Fall immer in die Bezugssteckkontaktöffnung gesteckt wird. Zu diesem Zweck ist der Bezugssteckkontakt 7E bezüglich der übrigen Steckkontaktöffnungen 7a, 7b, 7c und 7d bzw. der länglichen Steckkontaktöffnungen 7F und 7G wie gesagt getrennt, also nicht überlappend mit einer anderen Steckkontaktöffnung angeordnet. Durch den abgestimmten Abstand zwischen den Kontaktelementen des Steckverbinders und den Steckkontaktöffnungen der Universalsteckbuchse ergibt sich dann zwangsläufig die richtige Steckposition für das weitere Kontaktelement oder die weiteren Kontaktelemente des Steckverbinders. Auf diese Weise entfällt die Notwendigkeit einer zusätzlichen Steckpositionskodierung, beispielsweise in Form einer Asymmetrie oder einer Nase vollständig.

Insgesamt zeigt sich somit, dass die in Fig. 4 dargestellte Universalsteckbuchse 7 gemäß der Erfindung durch die überlappende Anordnung von Steckkontaktöffnungen und weiterhin durch die Ausbildung einer gemeinsamen Bezugssteckkontaktöffnung 7E für unterschiedliche Steckverbinder so ausgebildet ist, dass sie Steckverbinder sowohl für monopolare elektrochirurgische Instrumente als auch für bipolare elektrochirurgische Instrumente aufnehmen kann.

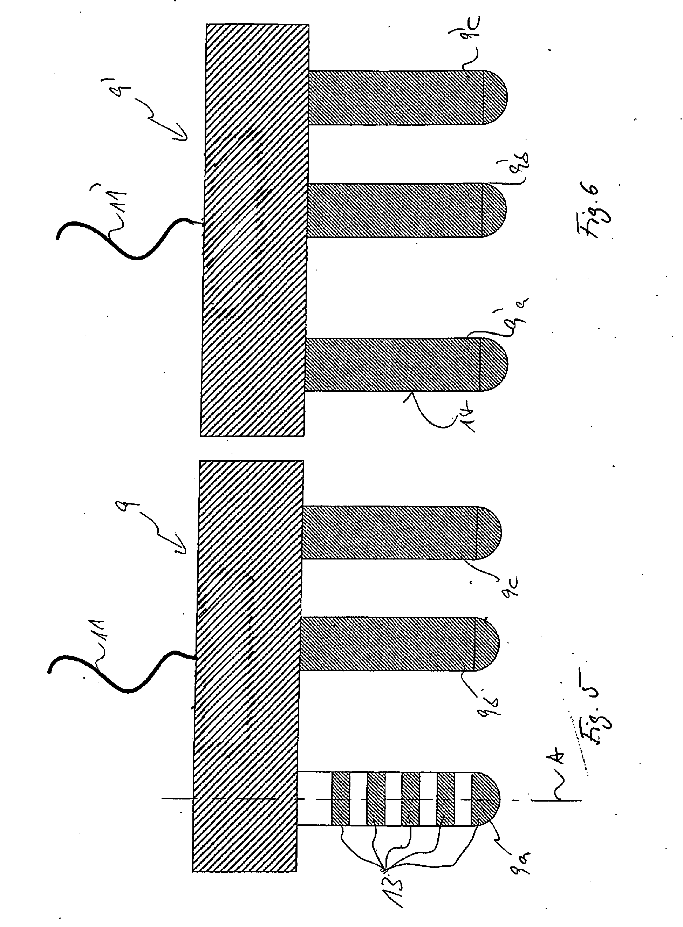

Fig. 5 zeigt eine schematische Seitenansicht eines Steckverbinders 9, der über eine Anschlussleitung 11 mit einem in der Figur nicht gezeigten elektrochirurgischen Instrument zur Übertragung eines hochfrequenten Stroms verbunden ist. Der Steckverbinder 9 weist zur Verbindung mit einer Steckbuchse drei Kontaktstifte 9a, 9b und 9c auf und sorgt so für eine Verbindung zwischen einem Chirurgiegerät und einem monopolaren elektrochirurgischen Instrument. Eine zugehörige Steckbuchse nach dem Stand der Technik ist in Fig. 1 gezeigt.

Der Steckverbinder 9 ist als multifunktionaler Instrumentenstecker ausgebildet. Hierzu ist das Kontaktelement 9a mit mehreren voneinander elektrisch getrennten ringförmigen Kontaktbereichen bzw. Kontaktflächen 13 versehen, die in einer axialen Richtung entlang einer Mittelachse A des Kontaktelements 9a auf dessen Umfangsfläche ringförmig in Abständen angeordnet sind. Die Kontaktflächen 13 sind mit entsprechenden Kabeln im Inneren des Kontaktelements verbunden, die wiederum über die Anschlussleitung 11 zu dem in der Figur nicht gezeigten elektrochirurgischen Instrument geführt werden.

Die ringförmigen Kontaktflächen 13 ermöglichen weitere Funktionalitäten des Steckverbinders 9 bzw. des elektrochirurgischen Instruments, wie beispielsweise eine Instrumentenerkennung oder eine Aktivierungserkennung, die weiter unten noch näher erläutert wird.

In der Fig. 6 ist ein Steckverbinder 9' dargestellt, der ebenfalls drei Kontaktelemente 9a', 9b' und 9c' aufweist, und der ebenfalls über ein Verbindungskabel 11' mit einem entsprechenden monopolaren elektrochirurgischen Instrument verbunden ist. Im Gegensatz zu dem Steckverbinder 9 gemäß Fig. 5 ist der Steckverbinder 9' jedoch nicht als multifunktionaler Steckverbinder ausgebildet, ermöglicht also keine Zusatzfunktionen. Das Kontaktelement 9a' bildet folglich auch keine ringförmigen, elektrisch voneinander getrennten Kontaktflächen aus, sondern umfasst eine einzige durchgehende Kontaktfläche 14, die in eine entsprechende Steckkontaktöffnung der Steckbuchse gesteckt wird.

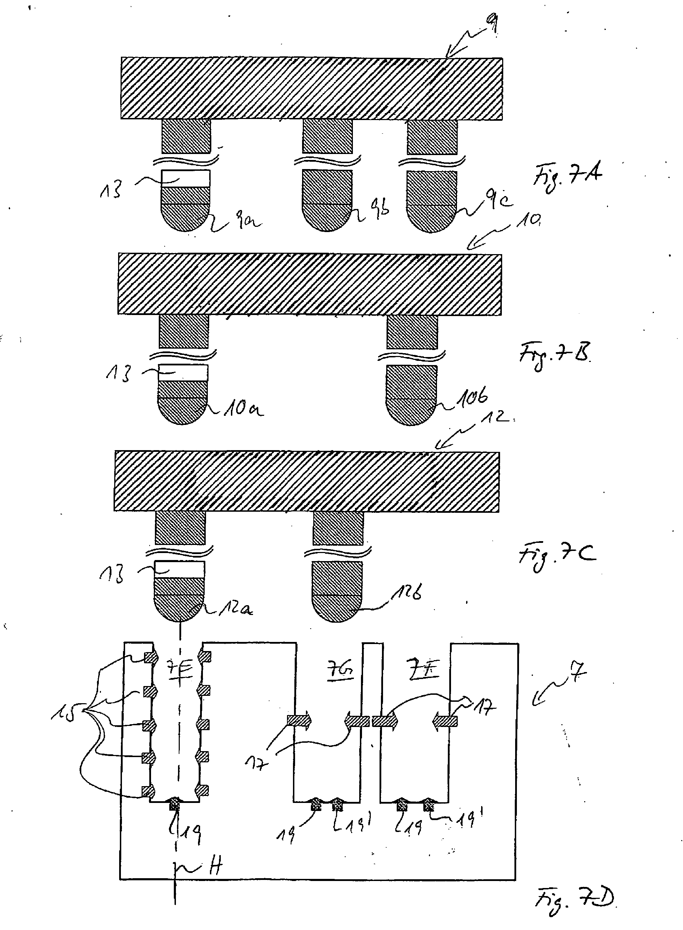

Die Fig. 7A bis 7C zeigen die verschiedenen beispielhaften Steckverbindertypen, die mit einer Universalsteckbuchse gemäß der vorliegenden Erfindung (Fig. 7D) verbindbar sind. Die dargestellten Positionen der Steckverbinder in den Fig. 7A bis 7C entsprechen dabei den korrekten Steckpositionen in der Universalsteckbuchse gemäß Fig. 7D.

Fig. 7A zeigt wiederum den Steckverbinder 9, der entweder einfach mit einer einzigen Kontaktfläche 14 oder multifunktional mit mehreren Kontaktflächen 13 an mindestens einem Kontaktelement ausgebildet sein kann. Auch die Steckverbinder 10 und 12 mit zwei Kontaktelementen 10a und 10b bzw. 12a und 12b gemäß Fig. 7B und Fig. 7C können entweder "einfach" mit einer einzigen Kontaktfläche oder multifunktional mit mehreren Kontaktflächen auf mindestens einem Kontaktelement ausgebildet sein. Die Fig. 7A bis 7C machen deutlich, dass im vorliegenden Beispiel die mehreren ringförmigen Kontaktflächen 13 vorzugsweise auf demjenigen Kontaktelement 9a, 10a und 12a ausgebildet sind, der in die Bezugssteckkontaktöffnung 7E eingeführt wird ("Bezugskontaktstift"). Es versteht sich, dass die mehreren Kontaktflächen 13 auch auf jedem anderen Kontaktelement oder auch auf mehreren Kontaktelementen angeordnet sein können.

Die Fig. 7D macht deutlich, dass die länglichen Steckkontaktöffnungen 7F und 7G, die also aus zwei überlappenden herkömmlichen Steckkontaktöffnungen bestehen, die Möglichkeit eröffnen, Steckverbinder sowohl für monopolare chirurgische Instrumente als auch für bipolare chirurgische Instrumente mit der Universalsteckbuchse 7 zu verbinden.

In der Fig. 7D ist die Bezugssteckkontaktöffnung 7E zur Aufnahme eines multifunktionalen Steckverbinders ausgebildet, d.h. also zur Aufnahme eines Steckverbinders mit wenigstens einem mehrere Kontaktbereiche 13 aufweisenden Kontaktelement. Hierzu weist mindestens die Bezugssteckkontaktöffnung 7E an seiner Innenfläche mehrere Kontaktbereiche 15 auf, die vorzugsweise ringförmig ausgebildet und entlang einer Mittelachse H der Bezugssteckkontaktöffnung 7E in axialer Richtung elektrisch getrennt voneinander im Wandbereich der Bezugssteckkontaktöffnung 7E angeordnet sind und die im gesteckten Zustand eines multifunktionalen Steckverbinders, wie beispielsweise der Steckverbinder 9 aus Fig. 5 mit den Kontaktbereichen 13 des Kontaktelements 9a in Kontakt kommen. Die Kontaktbereiche 15 sind mit Leitungen verbunden, die getrennte Strom- und/oder Signalwege bilden. Die Kontaktbereiche 15 können über die Leitungen beispielsweise mit einer Schaltmatrix und/oder mit einer Steuerungseinrichtung des Chirurgiegeräts verbunden sein.

Durch die mehreren Kontaktbereiche 15 in der Steckkontaktöffnung 7E können die mehreren Kontaktbereiche 13 auf dem Kontaktelement 9a kontaktiert werden, um eine erweiterte Funktionalität, wie beispielsweise eine Instrumentenerkennung oder eine Aktivierungserkennung des chirurgischen Instruments zu realisieren. Wird ein Standard-Instrument ohne einen Multifunktions-Steckverbinder mit einem einzigen Kontakt pro Kontaktelement in die Buchse eingesteckt, werden die mehrfachen Kontaktbereiche 15 in der Steckkontaktöffnung zumindest teilweise kurzgeschlossen und das chirurgische Instrument kann ohne die erweiterte Funktionalität wie gewohnt genutzt werden.

Fig. 7D macht noch deutlich, dass auch die Steckkontaktöffnungen 7F und 7G Kontaktflächen bzw. Kontaktbereiche 17 aufweisen, die zur Kontaktierung jeweils einer der Kontaktelemente der Steckverbinder dienen. Auch die Kontaktbereiche 17 der gemeinsamen Steckkontaktöffnungen 7F und 7G sind vorzugsweise entlang der Innenfläche der Steckkontaktöffnungen ringförmig angeordnet. Denkbar ist es jedoch auch getrennte Kontaktbereiche für jede einzelne Steckkontaktöffnung 7a, 7b, 7c und 7d vorzusehen, sodass also wenigstens zwei Kontaktbereiche pro gemeinsame Steckkontaktöffnung 7F bzw. 7G vorgesehen sind.

Darüber hinaus ist in der Schnittdarstellung der erfindungsgemäßen Universalsteckbuchse gemäß Fig. 7D zu erkennen, dass die Steckkontaktöffnungen 7F und 7G durch die überlappende Anordnung von jeweils zwei Steckkontaktöffnungen einen Querschnitt aufweisen, dessen Weite in Richtung der Mittelachse M größer ist als die Weite einer einzelnen Steckkontaktöffnung und insbesondere größer als die Bezugssteckkontaktöffnung 7E. Auf diese Weise können in die Steckkontaktöffnung 7F die Kontaktelemente 10b sowie 9c und in die Steckkontaktöffnung 7G die Kontaktelemente 12b und 9b wahlweise eingeführt werden.

Zusätzlich zu der erfindungsgemäßen mechanischen Kompatibilität mit einer Vielzahl von marktüblichen Steckverbindern, kann eine Vorrichtung in die Universalsteckbuchse integriert werden, welche das Vorhandensein der einzelnen Kontaktelemente der Steckverbinder im gesteckten Zustand in den Steckkontaktöffnungen erkennen kann. Hierzu kann eine oder mehrere Steckerkennungseinrichtungen 19 und 19' insbesondere am Boden der Steckkontaktöffnungen oder auch an einer beliebigen anderen geeigneten Position in oder an den Steckkontaktöffnungen vorgesehen sein. Um zu verhindern, dass ein HF-Signal auf einen nicht dafür vorgesehenen Kontaktbereich abgegeben wird, muss eine eindeutige Zuordnung der vorhandenen Kontaktbereiche zu einer Instrumentenart gewährleistet werden. Dies kann durch eine elektrische oder elektromechanische Vorrichtung, wie beispielsweise zusätzliche Kontaktelemente, Mikroschalter oder ein Reed-Kontakt usw. erreicht werden.

Im Betrieb des Chirurgiegeräts kann die Funktionalität der Universalsteckbuchse wie folgt aussehen:
Eine Steuerungseinrichtung des HF-Chirurgiegeräts erhält eine Rückmeldung von der Steckerkennungseinrichtung 19 bzw. 19', z.B. in Form eines Signals, über den "Steckzustand" der Universalsteckbuchse, d.h. also insbesondere, dass ein Steckverbinder mit der Universalsteckbuchse verbunden ist und vorzugsweise auch welches elektrochirurgische Instrument mit dem Steckverbinder verbunden ist (monopolar, bipolar, Instrumententyp, Aktivierungszustand, etc....). Wird beispielsweise das Kontaktelement 12b des Steckverbinders 12 in der Steckkontaktöffnung 7G durch die Steckerkennungseinrichtung 19' erkannt, erfolgt eine Rückmeldung an die Steuerungseinrichtung, dass ein zwei-poliger bipolarer Steckverbinder (22,0 mm) mit der Universalsteckbuchse 7 verbunden ist, woraufhin ein entsprechender Strom vom HF-Generator an das elektrochirurgische Instrument abgegeben wird. Sofern noch eine Instrumentenerkennung vorgesehen ist, die insbesondere durch die multifunktionale Ausbildung einer Steckkontaktöffnung 7E mit mehreren Kontaktbereichen 15 und entsprechenden Kontaktbereichen 13 eines Kontaktelements 12a realisiert werden kann, ist eine exakte Anpassung der Stromparameter an ein bestimmtes elektrochirurgisches Instrument möglich.

Nach dem Erhalt der Rückmeldung von der Steckerkennungseinrichtung erzeugt die Steuerungseinrichtung des Chirurgiegeräts ein Signal, welches entsprechend der Rückmeldung über die Kontaktkonfiguration dafür sorgt, dass auf den passenden Kontaktelementen in den Steckkontaktöffnungen ein HF-Strom freigegeben wird. Einzelne Kontaktelemente können, je nach Instrument, sowohl zur Abgabe des HF-Stroms als auch als Signalleitung beispielsweise für eine Aktivierungserkennung eingesetzt werden. Bei multifunktionalen Instrumenten kann die Erkennung des Instrumententyps über elektrische Signale, beispielsweise von einem Widerstand, EEPROM, oder Mikrocontroller im Instrument, über die zusätzlichen in axialer Richtung voneinander getrennten ringförmigen Kontaktflächen realisiert werden.

Im vorliegenden Beispiel sind die multifunktionalen Kontaktelemente, d.h. die Kontaktstifte, die in axialer Richtung mehrere voneinander getrennte Kontaktflächen bzw. Kontaktbereiche aufweisen, nach Art eines Klinkensteckers ausgebildet. Denkbar ist es aber grundsätzlich auch, dass das mehrere Kontaktflächen aufweisende Kontaktelement mehrere elektrisch getrennte Kontaktpins aufweist, die sich in einer axialen Richtung parallel zueinander erstrecken. Es versteht sich, dass dann auch die zugehörige Steckkontaktöffnung entsprechend voneinander elektrisch getrennte Kontaktöffnungsbereiche bzw. Kontaktbuchsen zur Aufnahme der Kontaktpins aufweist, über die dann eine multifunktionale Betätigung des jeweiligen angeschlossenen elektrochirurgischen Instruments möglich ist.

Insgesamt zeigt sich, dass mit der erfindungsgemäßen Universalsteckbuchse 7 einerseits marktübliche monopolare und bipolare Steckverbinder verbunden werden können und andererseits eine Bezugssteckkontaktöffnung 7E vorgesehen ist, die eine eindeutige Steckposition für einen Steckverbinder vorgibt. Die Bezugssteckkontaktöffnung 7E in Verbindung mit den vorgegebenen Abständen der Kontaktelemente gewährleistet eine korrekte Positionierung des Steckverbinders in der Universalsteckbuchse. Durch den festen Kontaktabstand der Stifte untereinander eines jeden Steckverbinders bzw. Steckers ist damit eine eindeutige Steckposition garantiert. Es versteht sich, dass zusätzlich die Erkennung der einzigen Steckmöglichkeit dadurch realisiert werden kann, dass ein Bezugskontakt mit einer speziellen Form bzw. eine mechanische Codierung vorgesehen ist, wie beispielsweise eine gewisse Asymmetrie oder eine Nase. Als elektronische Lösung kann eine Schaltmatrix, eine Steuerung, eine Vorwahleinrichtung oder ein Datenspeicher vorgesehen sein. Weil jedes Steckersystem eine andere Kontaktbelegung haben kann, muss das Chirurgiegerät die Kontaktbelegung an der Universalsteckbuchse auf den aktuell eingesteckten Steckverbinder einstellen können, d.h. es ist eine Steckererkennung und eine Zuordnung des Steckers notwendig.

Sofern eine zusätzliche Funktionalität eines multifunktionalen Steckverbinders über ein Kontaktelement bzw. eine Steckkontaktöffnung realisiert wird kann dieser vorzugsweise bis zu 8 oder mehr Kontaktelemente in Form von Kontaktstiften aufweisen.

Die erfindungsgemäße Steckbuchse kann weitere auch zusätzliche Kontaktelemente bzw. Kontaktmittel zur Übertragung elektrischer Signale bzw. Daten aufweisen. Diese Kontaktmittel können in beliebiger Form beispielsweise in Form eines USB (Universial Serial Bus)-Kontaktes ausgebildet sein.

Es wird eine Universalsteckbuchse 7 für ein Chirurgiegerät zum Anschluss von unterschiedlichen Steckverbindern 9, 9', 10, 12 verschiedener chirurgischer Instrumente für die Hochfrequenz-Chirurgie, aufweisend mehrere Steckkontaktöffnungen 7a, 7b, 7c, 7d, 7E zur Aufnahme von Kontaktelementen 9a, 9a', 9b, 9b', 9c, 9c', 10a, 10b, 12a, 12b eines Steckverbinders 9, 9', 10, 12 vorgeschlagen. Die Universalsteckbuchse 7 zeichnet sich dadurch aus, dass zum wahlweisen Anschluss von monopolaren und bipolaren chirurgischen Instrumenten mehrere Steckkontaktöffnungen 7a, 7b, 7c, 7d überlappend angeordnet sind, und dass zur Festlegung einer eindeutigen Steckposition eines Steckverbinders 9, 9', 10, 12 eine gemeinsame Bezugssteckkontaktöffnung 7E für unterschiedliche Steckverbinder vorgesehen ist, die bezüglich der anderen Steckkontaktöffnungen 7a, 7b, 7c, 7d getrennt angeordnet ist.

### Bezugszeichenliste

- 1: Steckbuchse
- 1a, b, c: Steckkontaktöffnungen
- 3: Steckbuchse
- 3a, 3b: Steckkontaktöffnungen
- 5: Steckbuchse
- 5a, 5b: Steckkontaktöffnungen
- 7: Universalsteckbuchse
- 7a, b, c, d: Steckkontaktöffnungen
- 7G, F: Längliche, bzw. ovale Steckkontaktöffnungen
- 7E: Bezugssteckkontaktöffnung
- 9, 9': Steckverbinder
- 9a, b, c: Kontaktelement, Kontaktmittel
- 10: Steckverbinder
- 11, 11': Anschlussleitung
- 12: Steckverbinder
- 13: Kontaktbereiche
- 14: Kontaktfläche
- 15: Kontaktbereiche
- 17: Kontaktflächen
- 19, 19': Steckerkennungseinrichtung
- M: Mittelachse Universalsteckbuchse
- A: Mittelachse Kontaktelement
- H: Mittelachse Bezugssteckkontaktöffnung

## Patentansprüche

1. Universalsteckbuchse (7) für ein Chirurgiegerät zum Anschluss von unterschiedlichen Steckverbindern (9, 9', 10, 12) verschiedener chirurgischer Instrumente für die Hochfrequenz-Chirurgie, aufweisend mehrere Steckkontaktöffnungen (7a, 7b, 7c, 7d, 7E) zur Aufnahme von Kontaktelementen (9a, 9a', 9b, 9b', 9c, 9c', 10a, 10b, 12a, 12b) eines Steckverbinders (9, 9', 10, 12),
**dadurch gekennzeichnet, dass**
zum wahlweisen Anschluss von monopolaren und bipolaren chirurgischen Instrumenten mehrere Steckkontaktöffnungen (7a, 7b, 7c, 7d) überlappend angeordnet sind, und dass zur Festlegung einer eindeutigen Steckposition eines Steckverbinders (9, 9', 10, 12) eine gemeinsame Bezugssteckkontaktöffnung (7E) für unterschiedliche Steckverbinder vorgesehen ist, die bezüglich der anderen Steckkontaktöffnungen (7a, 7b, 7c, 7d) getrennt angeordnet ist.

2. Universalsteckbuchse nach Anspruch 1,
**dadurch gekennzeichnet, dass**
zumindest eine Steckkontaktöffnung (7E) mehrere elektrisch getrennte Kontaktbereiche (15) zur Kontaktierung eines Kontaktelementes aufweist.

3. Universalsteckbuchse nach Anspruch 2,
**dadurch gekennzeichnet, dass**
die mehreren elektrisch getrennten Kontaktbereiche (15) der zumindest einen Steckkontaktöffnung (7E) zur Kontaktierung eines Kontaktelementes (9a, 10a, 12a) ausgebildet sind, das nach Art eines Klinkensteckers ausgebildet ist und mehrere in axialer Richtung des Kontaktelementes (9a, 10a, 12a) elektrisch getrennte Kontaktbereiche (13) aufweist.

4. Universalsteckbuchse nach einem der vorhergehenden Ansprüche,
**dadurch gekennzeichnet, dass**
sie wenigstens eine Steckerkennungseinrichtung (19, 19') zur Erkennung eines in der Universalsteckbuchse (7) angeordneten Steckverbinders (9, 10, 12) aufweist.

5. Universalsteckbuchse nach Anspruch 4,
**dadurch gekennzeichnet, dass**
die wenigstens eine Steckerkennungseinrichtung (19, 19') durch wenigstens einen zusätzlichen Kontaktpin, durch wenigstens einen Reedkontakt oder durch wenigstens einen Mikroschalter realisiert ist.

6. Universalsteckbuchse nach einem der vorhergehenden Ansprüche,
**dadurch gekennzeichnet, dass**
eine Instrumentenerkennungseinrichtung zur Erkennung des mit der Universalsteckbuchse (7) verbundenen chirurgischen Instrumententyps vorgesehen ist.

7. Universalsteckbuchse nach einem der vorhergehenden Ansprüche,
**dadurch gekennzeichnet, dass**
wenigstens zwei Steckkontaktöffnungen (7c, 7E) vorgesehen sind, die einen Abstand von 22,0 mm zueinander aufweisen.

8. Universalsteckbuchse nach einem der vorhergehenden Ansprüche,
**dadurch gekennzeichnet, dass**
wenigstens zwei Steckkontaktöffnungen (7b, 7E) vorgesehen sind, die einen Abstand von 28,5 mm zueinander aufweisen.

9. Universalsteckbuchse nach einem der vorhergehenden Ansprüche,
**dadurch gekennzeichnet, dass**
der Querschnitt einer Steckkontaktöffnung (7G, 7E), die aus zwei überlappenden Steckkontaktöffnungen (7a, 7b; 7c, 7d) gebildet ist, vom Querschnitt der Steckkontaktöffnung (7E) abweicht.

10. Universalsteckbuchse nach Anspruch 9,
**dadurch gekennzeichnet, dass**
die Weite des Querschnitts der Steckkontaktöffnung (7G, 7E), gemessen in Längsrichtung der Mittelachse (M) größer ist als die Weite der Steckkontaktöffnung (7E).

11. Steckverbindung umfassend eine Universalsteckbuchse (7) gemäß Anspruch 1 und wenigstens ein Steckverbinder (9, 9', 10, 12) der wenigstens zwei Kontaktelemente (9a, 9a', 9b, 9b', 9c, 9c', 10a, 10b, 12a, 12b) aufweist.

12. Steckverbindung nach Anspruch 11,
**dadurch gekennzeichnet, dass**
die Steckverbindung wenigstens ein Kontaktmittel, das in Form eines Kontaktstifts und wenigstens ein weiteres Kontaktmittel, das in Form einer Buchse ausgebildet ist, aufweist, und dass sowohl der Kontaktstift wie auch die Kontaktbuchse mehrere elektrisch getrennten Kontaktbereiche (15) aufweisen, wobei die Kontaktbuchse der Steckkontaktöffnung (7E) und der Kontaktstift dem Kontaktelement (9a, 10a, 12a) zugeordnet ist.

## Claims

1. Universal plug socket (7) for a surgical device for connecting different plug connectors (9, 9', 10, 12) of a variety of surgical instruments for radio-frequency surgery, having a plurality of plug contact openings (7a, 7b, 7c, 7d, 7E) for receiving contact elements (9a, 9a', 9b, 9b', 9c, 9c', 10a, 10b, 12a, 12b) of a plug connector (9, 9', 10, 12),
**characterized in that**
a plurality of plug contact openings (7a, 7b, 7c, 7d) are arranged in an overlapping manner for selectively connecting monopolar and bipolar surgical instruments, and **in that**, for the purpose of defining an unambiguous plug position of a plug connector (9, 9', 10, 12), a common reference plug contact opening (7E) is provided for different plug connectors, said reference plug contact opening being arranged separately from the other plug contact openings (7a, 7b, 7c, 7d).

2. Universal plug socket according to Claim 1,
**characterized in that**
at least one plug contact opening (7E) has a plurality of electrically separated contact regions (15) for making contact with a contact element.

3. Universal plug socket according to Claim 2,
**characterized in that**
the plurality of electrically separate contact regions (15) of the at least one plug contact opening (7E) is designed for making contact with a contact element (9a, 10a, 12a) which is designed in the manner of a jack connector and has a plurality of contact regions (13) which are electrically separated in the axial direction of the contact element (9a, 10a, 12a).

4. Universal plug socket according to one of the preceding claims,
**characterized in that**
it has at least one plug identification device (19, 19') for identifying a plug connector (9, 10, 12) which is arranged in the universal plug socket (7).

5. Universal plug socket according to Claim 4,
**characterized in that**
the at least one plug identification device (19, 19') is realized by at least one additional contact pin, by at least one reed contact or by at least one microswitch.

6. Universal plug socket according to one of the preceding claims,
**characterized in that**
an instrument identification device is provided for identifying the type of surgical instrument which is connected to the universal plug socket (7).

7. Universal plug socket according to one of the preceding claims,
**characterized in that**
at least two plug contact openings (7c, 7E) are provided, which are at a distance of 22.0 mm from one another.

8. Universal plug socket according to one of the preceding claims, **characterized in that**
at least two plug contact openings (7b, 7E) are provided, which are at a distance of 28.5 mm from one another.

9. Universal plug socket according to one of the preceding claims,
**characterized in that**
the cross section of a plug contact opening (7G, 7F) which is formed from two overlapping plug contact openings (7a, 7b; 7c, 7d) differs from the cross section of the plug contact opening (7E).

10. Universal plug socket according to Claim 9,
**characterized in that**
the width of the cross section of the plug contact opening (7G, 7F), measured in the longitudinal direction of the centre axis (M), is greater than the width of the plug contact opening (7E).

11. Plug connection comprising a universal plug socket (7) according to Claim 1 and at least one plug connector (9, 9', 10, 12) which has at least two contact elements (9a, 9a', 9b, 9b', 9c, 9c', 10a, 10b, 12a, 12b).

12. Plug connection according to Claim 11,
**characterized in that**
the plug connection has at least one contact means which is designed in the form of a contact pin, and at least one further contact means which is designed in the form of a socket, and **in that** both the contact pin and also the contact socket have a plurality of electrically separated contact regions (15), with the contact socket being associated with the plug contact opening (7E) and the contact pin being associated with the contact element (9a, 10a, 12a).

## Revendications

1. Fiche femelle universelle (7) conçue pour un appareil chirurgical en vue de raccorder différents connecteurs enfichables (9, 9', 10, 12) de différents instruments chirurgicaux dans le domaine de la chirurgie haute fréquence, comportant plusieurs ouvertures de contact d'enfichage (7a, 7b, 7c, 7d, 7E) permettant de recevoir les éléments de contact (9a, 9a', 9b, 9b', 9c, 9c', 10a, 10b, 12a, 12b) d'un connecteur enfichable (9, 9', 10, 12), **caractérisée en ce que** plusieurs ouvertures de contact d'enfichage (7a, 7b, 7c, 7d) sont disposées de façon à se chevaucher en vue de raccorder au choix des instruments chirurgicaux monopolaires ou bipolaires et qu'une ouverture de contact d'enfichage de référence (7E) commune est prévue pour différents connecteurs enfichables en vue de déterminer une position d'enfichage univoque pour un connecteur enfichable (9, 9', 10, 12), ladite ouverture étant disposée de façon séparée par rapport aux autres ouvertures de contact d'enfichage (7a, 7b, 7c, 7d).

2. Fiche femelle universelle selon la revendication 1, **caractérisée en ce qu'**au moins une ouverture de contact d'enfichage (7E) comporte plusieurs zones de contact (15) séparées sur le plan électrique servant à réaliser la mise en contact d'un élément de contact.

3. Fiche femelle universelle selon la revendication 2, **caractérisée en ce que** les multiples zones de contact (15) séparées sur le plan électrique de l'au moins une ouverture de contact d'enfichage (7E) sont réalisées pour mettre en contact un élément de contact (9a, 10a, 12a) prenant la forme d'une fiche mâle à cliquet et comportant plusieurs zones de contact (13) séparées sur le plan électrique dans la direction axiale de l'élément de contact (9a, 10a, 12a).

4. Fiche femelle universelle selon l'une quelconque des revendications précédentes, **caractérisée en ce qu'**elle comporte au moins un dispositif de détection d'enfichage (19, 19') permettant de détecter un connecteur enfichable (9, 10, 12) disposé dans la fiche femelle universelle (7).

5. Fiche femelle universelle selon la revendication 4, **caractérisée en ce que** l'au moins un dispositif de détection d'enfichage (19, 19') est réalisé par le biais d'au moins une broche de contact supplémentaire, d'au moins un contact à lames souples ou d'au moins un microcontacteur.

6. Fiche femelle universelle selon l'une quelconque des revendications précédentes, **caractérisée en ce qu'**un dispositif de détection d'instrument est prévu pour détecter le type d'instrument chirurgical relié à la fiche femelle universelle (7).

7. Fiche femelle universelle selon l'une quelconque des revendications précédentes, **caractérisée en ce qu'**au moins deux ouvertures de contact d'enfichage (7c, 7E) sont prévues et que lesdites ouvertures sont espacées de 22,0 mm les unes par rapport aux autres.

8. Fiche femelle universelle selon l'une quelconque des revendications précédentes, **caractérisée en ce qu'**au moins deux ouvertures de contact d'enfichage (7b, 7E) sont prévues et que lesdites ouvertures sont espacées de 28,5 mm les unes par rapport aux autres.

9. Fiche femelle universelle selon l'une quelconque des revendications précédentes, **caractérisée en ce que** la section transversale d'une ouverture de contact d'enfichage (7G, 7F) constituée de deux ouvertures de contact d'enfichage (7a, 7b ; 7c, 7d) se chevauchant s'écarte de la section transversale de l'ouverture de contact d'enfichage (7E).

10. Fiche femelle universelle selon la revendication 9, **caractérisée en ce que** la largeur de la section transversale de l'ouverture de contact d'enfichage (7G, 7F) mesurée dans la direction longitudinale de l'axe central (M) est supérieure à la largeur de l'ouverture de contact d'enfichage (7E).

11. Liaison par enfichage comprenant une fiche femelle universelle (7) selon la revendication 1 et au moins un connecteur enfichable (9, 9', 10, 12) comportant au moins deux éléments de contact (9a, 9a', 9b, 9b', 9c, 9c', 10a, 10b, 12a, 12b).

12. Liaison par enfichage selon la revendication 11, **caractérisée en ce que** la liaison par enfichage comporte au moins un moyen de contact prenant la forme d'une tige de contact et au moins un moyen de contact supplémentaire prenant la forme d'une fiche femelle et que tant la tige de contact que la fiche femelle de contact comportent plusieurs zones de contact (15) séparées sur le plan électrique, la fiche femelle de contact étant associée à l'ouverture de contact d'enfichage (7E) et la tige de contact étant associée à l'élément de contact (9a, 10a, 12a).
